# EUROPEAN PATENT SPECIFICATION

(11) **EP 0 543 988 B1**
(45) Date of publication and mention of the grant of the patent: **21.06.1995**
(21) Application number: 92914163.8
(22) Date of filing: 15.06.1992
(51) Int. Cl.: A61K 47/02, A61K 49/00, A61M 36/14, B03C 1/30, B01D 35/06, C08B 37/02, C02F 1/48, C07H 15/00, C07H 17/00, C07H 23/00

(54) **PROCESS FOR PRODUCING MAGNETICALLY RESPONSIVE POLYMER PARTICLES AND APPLICATION THEREOF**
Verfahren zur Herstellung magnetisch empfindlicherPolymerpartikel und ihre Anwendung
PROCEDE DE PRODUCTION DE PARTICULES POLYMERES A SENSIBILITE MAGNETIQUE, ET APPLICATION DE CELUI-CI

(30) Priority: 17.06.1991 US 716144
(43) Date of publication of application: 02.06.1993
(73) Proprietor: DADE INTERNATIONAL INC., Deerfield, Illinois 60015-0778 (US)
(72) Inventor: WANG, Chao-Huei, J., Gurnee, IL 60031 (US); SHAH, Dinesh, O., Vernon Hills, IL 60061 (US)
(74) Representative: MacGregor, Gordon
(86) International application number: US9204995
(87) International publication number: WO9222201

(56) References cited:
- US-A- 4 177 253
- US-A- 4 490 436
- US-A- 4 935 147
- US-A- 4 985 233

## Description

### Field of the Invention

This invention relates to a process to make magnetically responsive polymer particles and their use in immunoassays, biomedical and industrial applications.

### Background of the Invention

Many biological techniques, such as immunoassays, affinity purification, etc., require the separation of bound from free fractions. Magnetic particles have been used to facilitate the desired separation.

Magnetic particles have been formed from a variety of particulate and magnetic matter, using a variety of processes, having different characteristics. For example, Ikeda et al. U.S. Patent No. 4,582,622, discloses a magnetic particle comprised of gelatin, water-soluble polysaccharide, sodium phosphate and ferromagnetic substances; U.S. Patent Nos. 4,628,037 and 4,554,088 disclose magnetic particles comprised of a magnetic metal oxide core surrounded by a coat of polymeric silane; U.S. Patent No. 4,452,773 discloses discrete colloidal sized particles having a core of ferromagnetic iron oxide (Fe₃O₄) which is coated with a water-soluble polysaccharide or a derivative thereof having functional groups; Mansfield US Patent No. 4,297,337 discloses magnetic glass- or crystal-containing material as a particulate carrier.

US-A-4490436 discloses a particle comprising a filler-containing polymer core particle which is coated with a filler-free polymer layer. The filler is a metal oxide. The filler is treated in order to make it oleophilic before it is used in the core particle. This treatment facilitates even dispersal of the filler in the core polymer, US-A-4490436 teaches that filler agglomeration is undesirable.

US-A-4177253 discloses a magnetic particle comprising a polymeric core having a magnetic material, e.g. iron oxide evenly coated thereon by electroless deposition.

US-A-4985233 discloses macromelecular particles made from a swellable, gelatinous polymeric network. A paramagnetic species is either chemically bound to the particle (e.g. by chelation), or included in cavities of the particle. There is no disclosure of an external polymeric layer around the surface of the macromelecular particle.

US-A-4935147 discloses a particle separation method using paramagnetic particles of a metal oxide, which may optionally have surface functional groups and/or a polysaccharide or protein coating. Also disclosed is a particle comprising polymer matrix in which is impregnated the metal oxide. The latter particles are, however, not preferred, because they tend to be too large for performing the disclosed method.

The precharacterising part of Claim 1 is based on US-A-4177253, and is directed to a paramagnetic particle comprising an inner polymeric core particle 1 to 100 microns in diameter and an external layer containing metal oxide crystals. The distinguishing features of the present invention are set out in the characterising part of Claim 1, and are characterised in that said layer is a polymeric layer polymerizer at the surface of said core particle, the metal oxide crystals being agglomerated with an amorphous metal oxide precipitate, into nonuniformly sized clusters of about 1.0 microns or less

In a process of producing magnetically responsive polymer particles, hereinafter referred to as magnetic particles, from polymeric particles with average size from about 1 to 100 microns in diameter regardless of shape and composition, a magnetically responsive metal oxide is first produced, hereinafter referred to as metal oxide, with average size of about 1 micron or less. A polymeric core particle is then coated with a layer of polymer containing metal oxide. The surface of these magnetic particles can be coated further with another layer of polymer or functionalized polymer to provide the desired surface characteristics.

The magnetic particles are monodispersed in size with rough surface and have a magnetic metal oxide content of from about 5% to 50%, preferably from 10% to 25%. Particles with these characteristics have been found to be useful in immunoassays and a wide variety of biomedical applications. These magnetic particles can be used for passive or covalent coupling of biological material such as antigens, antibodies, enzymes or DNA/RNA and used as solid phase for various types of immunoassays, DNA/RNA hybridization assays, affinity purification, cell separation and other biomedical applications. The magnetic particles can also be used for industrial application such as the treatment of industrial waste.

It is the objective of this invention to:
Develop magnetically responsive polymer particles which are easily produced from readily available polymer particles. Hence,
a wide variety of polymeric core particles with size from about 1 to 100 microns can easily be transformed to magnetically responsive particles.

The metal oxide content can be varied according to the applications.

The surface can be derivatized into a wide variety of functional groups for covalent coupling.

A wide variety of monomer can be used for the final coating to provide different surface characteristics of the resulting polymer.

Both cross-linked and noncross-linked magnetically responsive polymer particles can be produced.

Monodispersed magnetically responsive polymer particles can be produced.

### Detailed Description of the Invention

The magnetic particles of this invention may be prepared by first producing metal oxide with average size of about 1 micron or less. The metal oxide is produced by heating and precipitating a mixture of divalent and trivalent metal salt, preferably a mixture of ferrous and ferric sulfate or chloride with sodium hydroxide solution. The molar ratio of divalent to trivalent metal salt can be varied from 0.5 to 2.0, preferably 0.5 to 1.0, to obtain the desirable size and magnetic characteristics of metal oxide. It is observed that the molar ratio of divalent to trivalent metal salt affects the size of the metal oxide: the smaller the molar ratio of divalent to trivalent metal salt, the smaller the size of metal oxide. The molar ratio of divalent to trivalent metal salt also affects the color of the resulting magnetic particles: the smaller the molar ratio, the lighter the brownish color of the resulting magnetic particles. Preferable, the metal oxide is either superparamagnetic or paramagnetic although ferromagnetic metal oxide can also be used, provided centrifugation instead of magnetic separation is used during the clean up. Other divalent transition metal salts such as manganese, magnesium, cobalt, nickel, zinc, and copper salts may be substituted for ferrous salt.

After the metal oxide has been precipitated, it is washed several times with centrifugation at 250 xg until the supernatant is neutral in pH. The metal oxide is resuspended in deionized water and mechanically stirred at high speed to break down the aggregate of metal oxide crystals. Further centrifugation at 250 xg will not pellet all of the metal oxide. The supernatant which contain smaller size metal oxide crystals is collected and the pellet is resuspended in deionized water. This process is repeated for at least three times or until most of metal oxide can no longer be pelleted at 250 xg. The metal oxide obtained this way usually has size less than 2.0 micron. Low speed centrifugation at 100 xg to remove larger crystals will reduce the size to less than 0.8 micron.

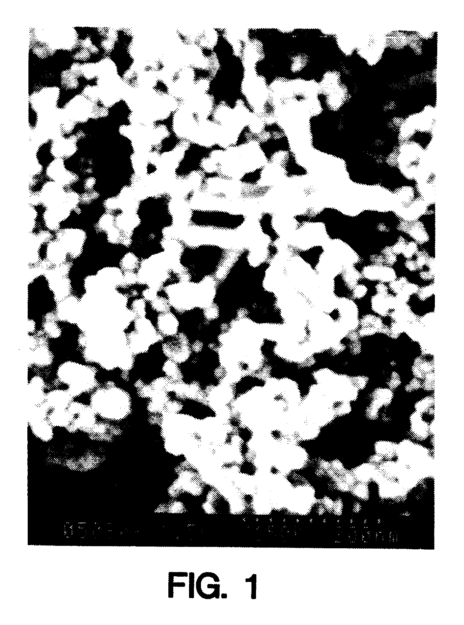

Upon examination by electron microscopy as shown in Figure 1, it is evident that supernatant metal oxide crystals are still partially aggregated, even though they have been stirred vigorously. It is a distinctive characteristic of the metal oxides produced by the instant method, that the smallest particles are agglomerated in clusters of small crystals with precipitated amorphous matter binding them together. The washing and stirring steps do not completely dissociate the particles, but leave them undissociated in clusters of agglomerated small crystals adhering together by amphorous matter. This appears to be critical to the coating step in preparing the final paramagnetic particles, since metal oxide control particles which are uniformly and completely dispersed, do not coat, but contribute to particle bridging causing severe aggregation.

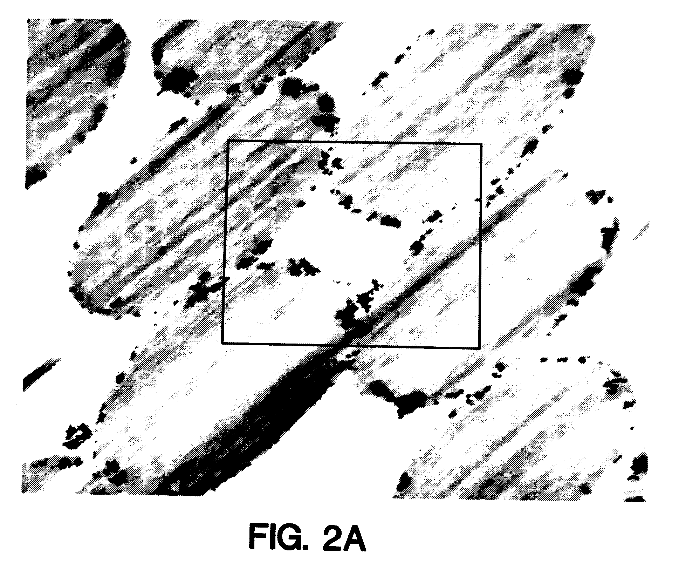

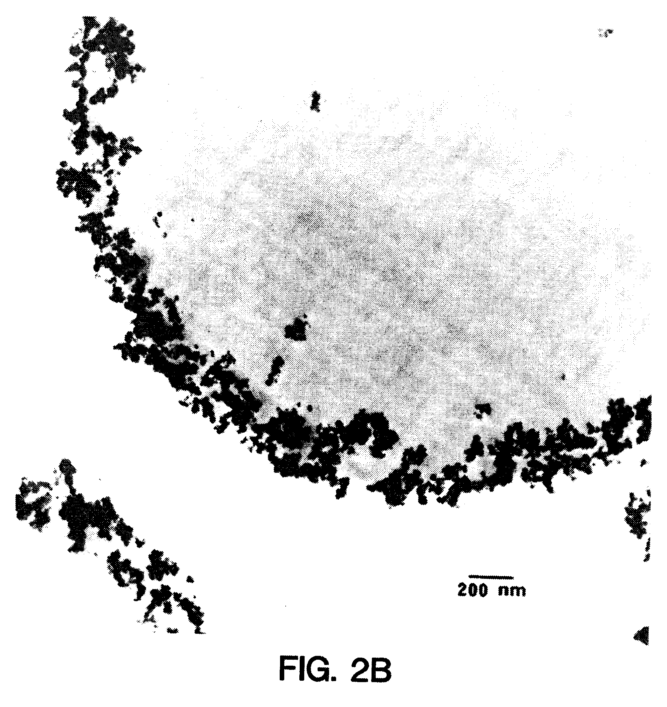

The metal oxide with average size of 1.0 micron or less is mixed with monomer and coated onto the polymeric core particles, preferably polystyrene particles, with size of 1 to 100 microns in the presence of initiator. Addition of a small quantity of emulsifier will help prevent the particles from agglomerating. Migration of the metal oxide cluster agglomerates occurs from the aqueous to the organic phase of the monomer undergoing polymerization at the surface of the polystyrene particle. The result is a distinctive uneven, nonuniformly distributed layer containing the metal oxide cluster agglomerates embedded in the polymer being laid down simultaneously at the surface of the particle. This layer is seen in the cross-sectional scanning electron micrographs shown in Figures 2a and 2b. Surprisingly, paramagnetic particles of this configuration have an unusual capacity for antigen-binding, compared to polystyrene particles of the same size without a paramagnetic layer, as demonstrated in the Examples. If functionalized magnetic particles are desired, the magnetic particles can be coated further with another layer of functionalized polymer to provide functional groups such as carboxyl, amino or hydroxyl for covalent coupling of biological material. Figure III shows a scanning electron micrograph of 6.8 micron magnetic particles, prepared according to this invention. Figure IIIa is at 1000x and Figure IIIb is at 5000x magnification.

The polymeric core particles useful in this invention may be of any polymer which can be obtained as a dispersion of small particles and which can absorb a monomer thereby causing the metal oxide and monomer mixture to coat onto the surface of the core particles. The core particles may be of any size and shape, preferable of 1 to 100 microns in size and spherical in shape. When monodispersed core particles are used the resulting magnetic particles will also be monodispersed in size. The core particles may be obtained by emulsion polymerization, suspension polymerization or other means of polymerization with or without a cross-linking agent such as divinyl benzene or the like. Among the monomers which can be used to prepare core particles are styrene, methyl methacrylate, vinyltoluene and the like. A mixture of the monomers can also be used. The monomer used for magnetic metal oxide coating or protective coating may or may not be the same type as the core particles. The weight ratio of monomer used for metal oxide coating to core particles may be from 0.1 to 12, preferably from 0.2 to 6, depending upon the thickness of metal oxide/polymer layer desired. When the metal oxide prepared from a mixture of ferrous and ferric salts is used for coating it is preferred to use a monomer to core particle weight ratio of about 0.1 to 0.5. However when the metal oxide prepared from a mixture of manganese (II) and ferric salts is used for coating the weight ratio of monomer to core particles may be from 0.1 to 12. As a result when cross-linked magnetic particles which are inert to common organic solvents are desired, it is preferred to use the metal oxide prepared from a mixture of manganese (II) and ferric salts with monomer containing 2% to 10%, preferably 8% to 10% by weight of cross-linking agent and a monomer to core particle weight ratio of 3 to 12, preferably 4 to 6. When lower monomer to core particle weight ratio (i.e., 0.1 to 0.5) is used during the metal oxide/polymer coating it is preferred to overcoat the resulting magnetic particles with a protective layer of polymer coating to further adhere the metal oxide to the surface of the magnetic particles. However, when higher monomer to core particle ratio (i.e., 3 to 12) is used no protective polymer coating is necessary. The polymerization temperature may be from 50^{o}C to 90^{o}C, preferably 55^{o}C to 65^{o}C. The polymerization initiator may either be water soluble such as potassium persulfate and the like or water insoluble such as benzoyl peroxide and the like. Other means of polymerization initiation such as radiation, ionization or the like may also be used. It is found unexpectedly that magnetic particles can be produced without using any emulsifier when the metal oxide prepared from a mixture of manganese (II) and ferric salts is used for coating. However, a small amount of emulsifier such as sodium dodecylsulfate, Aerosol 22, Tween 20 or Nonidet P-40 (NP 40) is found to be useful in preventing the particles from extensive aggregation during the metal oxide/polymer coating when the metal oxide prepared from a mixture of ferrous and ferric salts is used for coating. Other emulsifiers with the same capability may also be used. The magnetic metal oxide content can be varied from 5% to 50%, preferably from 10% to 25% by using different amounts of metal oxide during the metal oxide/polymer coating. Multiple metal oxide/polymer coating scan also be employed to increase the metal oxide content. Other ingredients commonly used in polymerization may also be added as long as magnetic particles with desirable characteristics can be obtained. The ingredients for metal oxide/polymer coating may be added all at once at the beginning of metal oxide/polymer coating process or added stepwise. When the metal oxide prepared from a mixture of ferrous and ferric salt is used, it is preferred to add the ingredients stepwise. The ingredients may be mixed by mechanic stirring, tumbling or other means of agitation under vacuum or inert gas such as argon. The functional groups can be incorporated onto the surface of the magnetic particles by either using a mixture of monomer and functionalized monomer during the metal oxide/polymer coating or overcoating the magnetic particles with a thin layer of functionalized monomer at the end. The functionalized monomer used may be selected from one or a mixture of the following: 2-hyroxyethyl methacrylate, 2-aminoethyl methacrylate, trimethylammoniumethyl methacrylate methosulfate, dimethylaminoethyl methacrylate, methacrylic acid, undecylenic acid, methyl propene sulfonic acid, undecylenyl alcohol, oleyl amine, glycidyl methacrylate, acrolein, glutaraldehyde and the like. The magnetic particles can also be overcoated with a layer of different polymer than the one used for metal oxide/polymer coating or protective coating to take up the surface characteristics of the polymer.

### Applications of Magnetic Particles

The uses of a wide variety of magnetic particles as solid phase for various applications such as fluorescence immunoassays, radioimmunoassays, enzyme immunoassays, cell separations, enzyme immobilizations and affinity purifications have been reviewed in literature as examplified by the following articles: Hirschbein et al., Chemical Technology, March 1982, 172-179 (1982); Pourfarzaneh, The Ligand Quarterly, 5(1):41-47 (1982); Halling and Dunnill, Enzyme Microbe Technology, 2:2-10 (1980); Mosbach and Anderson, Nature, 270:259-261 (1977); Guesdon et al., J. Allergy Clinical Immunology, 61(1), 23-27 (1978). Some applications have also been disclosed in the U.S. Patent Nos. 4,152,210 and 4,343,901 for enzyme immobilizations; U.S. Patent Nos. 3,970,518, 4,230,685, and 4,267,234 for cell separations; U.S. Patent Nos. 4,554,088, 4,628,037, and 3,933,997 for immunoassays.

Some magnetic particles may be useful in one application, but not in another application. For example, the magnetic particles disclosed in U.S. Patent Nos. 4,554,088 and 4,628,037, which comprise a superparamagnetic metal oxide core generally surrounded by a coat of polymeric silane, may be useful in immunoassay and affinity purification, due to the large surface area and slower settling rate, but are not suitable in cell separation application such as bone marrow purging. Due to the small size of the magnetic particles, disclosed in these two patents, it is very difficult to remove all of the magnetic particles from the cell suspension effectively. Moreover, the nonspecific binding of smaller magnetic particles to normal cells would be much higher. In using magnetic particles for bone marrow purging, the magnetic particles are coated with antibody, such as sheep anti-mouse IgG, and the bone marrow is treated with a mixture of several monoclonal antibodies against the cancer cell surface antigens. The magnetic particles will bind only to the cancer cells and cause them to be separated from normal cells by passing them through a strong magnetic field. The cleansed cells are then put back into the patient.

Magnetic particles according to the present invention can be optimized in terms of size, surface area, metal oxide content and surface characteristics for a wide variety of biomedical applications. The magnetic particles can be used as solid phase for enzyme immunoassay, fluorescence immunoassay, radioimmunoassay, DNA/RNA hybridization assay, and other diagnostic applications. Immunoassays can be performed by using various configurations such as sandwich assays and competitive binding assays etc., which are obvious to those skilled in the art. The DNA/RNA hybridization can also be performed by using various configurations such as solid phase hybridization or liquid phase hybridization. In solid phase hybridization configuration a DNA or RNA probe (catcher probe) is immobilized on the magnetic particle first. The immobilized catcher probe is then used to hybridize with complimentary strand of DNA from the sample (sample DNA). Finally another probe (signal probe) which is labelled with fluorescent, radioactive or enzyme tracer and capable of hybridizing with another part of the sample DNA is used for signal generation. In liquid phase hybridization configuration the catcher probe and signal probe are allowed to hybridize with the sample DNA in the liquid phase first and then immobilized to the magnetic particles.

Alternatively, the signal probe can also be labelled with one or several biotin groups and the signal is detected by binding the biotin groups with avidin labelled fluorescent, radioactive or enzymatic tracer to enhance the sensitivity of the assay.

The immunoassays and DNA/RNA hybridization assays can be used to measure a wide variety of compounds such as drugs, hormones, antibodies, peptides, DNA, RNA, nucleotides, viral antigens, and carbohydrates in biological samples.

The magnetic particles according to this invention can also be used for affinity purification, cell separation, enzyme immobilization and other biomedical applications. In cell separation the magnetic particles are used to either remove unwanted cells (negative selection) or enrich the wanted cells (positive selection) through immunological reactions or nonimmunological reactions. This principle can be used to remove cancer cells from bone marrow (bone marrow purging), purify cell populations through either positive or negative selection for tissue culture and perform various cellular immunoassays etc. In affinity purification the magnetic particles are used in place of conventional solid phase such as polyacrylamide gels, sepharose gels or other cellulose beads to purify a wide variety of biological materials such as antibodies, antigens, enzymes, inhibitors, cofactors, single stranded DNA, binding proteins, haptens and carbohydrates etc. In another application similar to the affinity purification, the magnetic particles can be used to cross adsorb and remove unwanted protein components from the antisera or clinical samples. In enzyme immobilization the enzyme is immobilized onto the magnetic particles through various means of coupling so as to preserve the enzyme activity and to permit the reuse of immobilized enzyme. The magnetic particles with immobilized enzyme can be used to replace other solid phases such as glass beads, controlled pore glass, silica gels and cellulose beads etc., which are commonly used in immobilized enzyme systems to produce wide variety of materials such as carbohydrates, amino acids, and proteins, etc.

The magnetic particles produced according to this invention can be used for industrial applications like the treatment of industrial waste, the remove harmful chemicals, i.e., organic or inorganic solvents from industrial material.

These applications are all facilitated by the ease of separation, fast reaction rate and large surface area common to most of magnetic particles. The following examples are provided to further illustrate the versatility and advantages of this invention. The details thereof are not to be construed as limitations, for it will be apparent that various equivalents, changes and modifications may be resorted to without departing from the spirit and scope thereof and it is understood that such equivalent embodiments are intended to be included therein.

### General Procedures for the Preparation of Metal Oxide

### Example 1

In a three-necked round bottom flask equipped with mechanical stirrer, condenser, thermometer, dropping funnel and heating mantle was placed a mixture containing 0.361 mol of ferrous sulfate and 0.369 mol of ferric sulfate (Fe⁺⁺/Fe⁺⁺⁺ ratio = 1) in 400 ml of deionized water. The mixture was heated to 85 to 90 ^{o}C with stirring and added dropwise 850 ml of 6 N sodium hydroxide over a period of 90 minutes. The mixture was stirred at 85 to 90 ^{o}C for one more hour and cooled to room temperature. The metal oxide precipitates were centrifuged at 250 xg for 10 minutes. The clear supernatant was decanted and the pellet was resuspended in 900 ml of deionized water using mechanical stirrer. This cleaning process was repeated six times or until the supernatant was almost neutral in pH. The supernatant was decanted and resuspended in 200 ml of deionized water. Further centrifugation at 250 xg will not pellet all of the metal oxide precipitates. The supernatant which contained smaller size metal oxide crystals was collected and the pellet was resuspended in 200 ml of deionized water. This process was repeated for at least three times or until most of metal oxide can no longer be pelleted at 250 xg. The metal oxide obtained this way usually has size less than 2.0 micron. The combined metal oxide suspension was centrifuged at 100 xg for 10 minutes. The supernatant was collected to give 800 ml of 8.6% w/v magnetic metal oxide suspension having the size less than 0.8 microns.

### Example 2

Same procedures as described in Example 1 were followed except 0.235 mol of ferrous sulfate, 0.297 mol of ferric sulfate (Fe⁺⁺/Fe⁺⁺⁺ ratio = 0.79) in 400 ml of deionized water and 480 ml of 6 N sodium hydroxide were used to give 2000 ml of 2.86% w/v suspension of magnetic metal oxide.

### Example 3

Same procedures as described in Example 1 were followed except 0.178 mol of ferrous sulfate, 0.298 mol of ferric sulfate (Fe⁺⁺/Fe⁺⁺⁺ ratio = 0.59) in 400 ml of deionized water and 520 ml of 6 N sodium hydroxide were used to give 1500 ml of 2.98% w/v suspension of magnetic metal oxide.

### Example 4

Same procedures as described in Example 1 were followed except 0.15 mol of ferrous sulfate, 0.276 mol of ferric sulfate (Fe⁺⁺/Fe⁺⁺⁺ ratio = 0.54) in 400 ml of deionized water and 520 ml of 6 N sodium hydroxide were used to give 700 ml of 6.88% w/v suspension of magnetic metal oxide.

### Example 5

Same procedures as described in Example 1 were followed except 0.116 mol of manganese sulfate, 0.146 mol of ferric sulfate (Mn⁺⁺/Fe⁺⁺⁺ ratio = 0.79) in 225 ml of deionized water and 240 ml of 6 N sodium hydroxide were used to give 1700 ml of 1.8% w/v suspension of magnetic metal oxide.

### Preparation of Magnetic Particles

### Example 6

A mixture containing 600 ml of deionized water, 6 ml of styrene and 80 ml of 8.6% w/v magnetic metal oxide prepared as described in Example 1, was placed in a sealed bottle. The bottle was evacuated and rotated at about 60 rpm in a 55^{o}C oven for one hour. To the mixture were added 12 g of potassium persulfate and 850 ml of 5% w/v, 4.0 micron polystyrene particles. The bottle was resealed, evacuated and rotated for one hour and added 50 ml of 2% sodium dodecylsulfate. After five more hours 6 ml of styrene and 10 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 1.6 liters with deionized water to give a 2.5% w/v suspension with about 11% magnetic metal oxide content and 4.3 micron average size.

### Example 7

The magnetic particles, 1.6 liters of 2.5% w/v, prepared as described in Example 6, were carboxylated by adding 1 g of sodium dodecylsulfate, 10 g of potassium persulfate and a solution containing 0.98 ml of undecylenic acid and 0.02 ml of divinyl banzene in 4 ml of methanol. The mixture was placed in a sealed bottle, evacuated and rotated at about 60 rpm in a 55^{o}C oven for five hours. The resulting carboxyl magnetic particles were separated magnetically and washed several times with deionized water until the supernatant was clear. The carrboxyl magnetic particles were resuspended to 680 ml with deionized water to give a 5.8% w/v suspension with about 11% magnetic metal oxide content and 4.3 micron average size.

### Example 8

A mixture containing 600 ml of deionized water, 6 ml of styrene and 80 ml of 8.6% w/v magnetic metal oxide prepared as described in Example 1, was placed in a sealed bottle. The bottle was evacuated and rotated at about 60 rpm in a 55^{o}C oven for one hour. To the mixture were added 12 g of potassium persulfate and 850 ml of 4.78% w/v, 6.1 micron polystyrene particles. The bottle was resealed, evacuated, rotated for five hours and added 6 ml of styrene and 10 g of potassium persulfate. The mixture was rotated for another fifteen hours, filtered through two layers of cheese cloth, separated magnetically and washed several times with deionized water until the supernatant was clear. The resulting magnetic particles were resuspended to 1.5 liters with deionized water and carboxylated by adding 1 g of sodium dodecylsulfate, 10 g of potassium persulfate and a solution containing 0.98 ml of undecylenic acid and 0.02 ml of divinyl benzene in 4 ml of methanol. The mixture was placed in a sealed bottle, evacuated and rotated at about 60 rpm in a 55^{o}C oven for five hours. The resulting carboxyl magnetic particles were separated magnetically and washed several times with deionized water until the supernatant was clear. The carboxyl magnetic particles were resuspended to 800 ml with deionized water to give a 4.3% suspension with about 11.6% magnetic metal oxide content and 6.8 micron average size.

### Example 9

A mixture containing 600 ml of deionized water, 6 ml of styrene and 60 ml of 8.6% w/v magnetic metal oxide prepared as described in Example 1, was placed in a three-necked round bottom flask and stirred at 67^{o}C for one hour under argon. To the mixture were added 12 g of potassium persulfate and 470 ml of 5% w/v, 2.7 micron polystyrene particles. The mixture was stirred at 67^{o}C for one hour and added 30 ml of 2% sodium dodecylsulfate. After stirring at 67^{o}C under argon for five more hours 6 ml of styrene and 6 g of potassium persulfate were added to the mixture. The mixture was stirred at 67^{o}C under argon for another fifteen hours, filtered through two layers of cheese cloth, separated magnetically and washed several times with deionized water until the supernatant was clear. The resulting magnetic particles were resuspended to 900 ml with deionized water and carboxylated by adding 0.6 g of sodium dodecylsulfate, 10 g of potassium persulfate and a solution containing 0.598 ml of undecylenic acid and 0.012 ml of divinyl benzene in 2.4 ml of methanol. The mixture was placed in a sealed bottle, evacuated and rotated at about 60 rpm in a 55^{o}C oven for five hours. The resulting carboxyl magnetic particles were separated magnetically and washed several times with deionized water until the supernatant was clear. The carboxyl magnetic particles were resuspended to 500 ml to give a 6.5% w/v suspension with about 14% magnetic metal oxide content and 4.0 micron average size.

### Example 10

A mixture containing 600 ml of deionized water, 6 ml of styrene and 60 ml of 8.6% w/v magnetic metal oxide prepared as described in Example 1, was placed in a sealed bottle. The bottle was evacuated and rotated at about 60 rpm in a 55^{o}C oven for one hour. To the mixture were added 12 g of potassium persulfate and 470 ml of 5% w/v, 2.7 micron polystyrene particles. The bottle was resealed, evacuated and rotated for one hour and added 30 ml of 2% sodium dodecylsulfate. After five more hours 6 ml of styrene and 10 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 500 ml with deionized water to give a 6.8% w/v suspension with about 14% magnetic metal oxide content and 4.0 micron average size.

### Example 11

A mixture containing 180 ml of deionized water, 2 ml of styrene and 20 ml of 8.6% w/v magnetic metal oxide, prepared as described in Example 1, was placed in a sealed bottle. The bottle was evacuated and rotated at about 60 rpm in a 55^{o}C oven for one hour. To the mixture were added 4 g of potassium persulfate and 160 ml of 6.8% w/v magnetic particles (3.0 micron, 14% metal oxide content), prepared as described in Example 10. The bottle was resealed, evacuated and rotated for one hour and added 10 ml of 2% sodium dodecylsulfate. After five more hours 2 ml of styrene and 2 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 160 ml with deionized water to give a 7.78% w/v suspension with about 19% metal oxide content and 4.2 micron average.

### Example 12

A mixture containing 90 ml of deionized water, 1 ml of styrene and 10 ml of 8.6% w/v magnetic metal oxide, prepared as described in Example 1, was placed in a sealed bottle. The bottle was evacuated and rotated at about 60 rpm in a 55^{o}C oven for one hour. To the mixture were added 1 g of potassium persulfate and 80 ml of 7.78% w/v magnetic particles (3.2 micron, 19% metal oxide content), prepared as described in Example 11. The bottle was resealed, evacuated and rotated for four hours and added 5 ml of 2% sodium dodecylsulfate. After five more hours 1 ml of styrene and 1 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 160 ml with deionized water to give a 4.5% w/v suspension with about 23% metal oxide content and 4.5 micron average size.

### Example 13

A mixture containing 400 ml of deionized water, 1.92 ml of styrene, 0.08 ml of divinyl benzene, 4 g of potassium persulfate, 20 g of 200-400 mesh 4% divinyl benzene cross-linked polystyrene beads and 10 ml of 8.6% w/v magnetic metal oxide, prepared as described in Example 1, was placed in a sealed bottle. The bottle was evacuated and rotated at about 60 rpm in a 55^{o}C oven for fifteen hours. The mixture was allowed to settle and the supernatant was decanted. The resulting magnetic beads were resuspended in 200 ml of deionized water and allowed to settle again. This process was repeated several times until the supernatant was clear. The resulting magnetic beads were resuspended in 200 ml of deionized water and added 0.1 g of sodium dodecylsulfate, 2.0 g of potassium persulfate, 0.48 ml of styrene, and 0.02 ml of divinyl benzene. The bottle was resealed, evacuated and rotated at about 60 rpm in a 55^{o}C oven for one hour and added a solution containing 0.098 ml of undecylenic acid and 0.002 ml of divinyl benzene in 0.4 ml of methanol. The mixture was rotated for four more hours and cleaned up by gravitational sedimentation as described previously. The water was removed by filtration and the carboxyl magnetic beads were dried to give 20 g of 200-400 mesh carboxyl magnetic beads.

### Example 14

A mixture containing 100 ml of deionized water, 0.5 ml of styrene, 2 g of potassium persulfate, 75 ml of 5% w/v 4.0 micron polystyrene particles and 10 ml of 6.88% w/v magnetic metal oxide, prepared as described in Example 4, was placed in a sealed bottle. The bottle was evacuated and rotated at about 60 rpm in a 55^{o}C oven for fifteen hours. The mixture was filtered through two layers of cheese cloth, separated magnetically and washed several times with deionized water until the supernatant was clear. The resulting magnetic particles were resuspended to 150 ml with deionized to give a 2.5% w/v suspension with about 14% metal oxide content and 4.3 micron average size.

### Example 15

Same procedure as described in Example 14 were followed except 20 ml of 6.88% w/v magnetic metal oxide, prepared as described in Example 4, was used to give 160 ml of 2.5% w/v suspension with about 18% metal oxide content and 4.3 micron average size.

### Example 16

A mixture containing 2000 ml of deionized water, 13 ml of styrene and 550 ml of 2.98% w/v magnetic metal oxide prepared as described in Example 3, was placed in a sealed bottle. The bottle was evacuated and rotated at about 60 rpm in 55^{o}C oven for one hour. To the mixture were added 20 g of potassium persulfate and 950 ml of 10% w/v, 3.0 micron polystyrene particles. The bottle was resealed, evacuated and rotated for one hour and added 60 ml of 2% sodium dodecylsulfate. After five more hours 8 ml of styrene and 10 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 3000 ml with deionized water to give a 3.38% w/v suspension with about 12% magnetic metal oxide content and 3.2 micron average size.

### Example 17

A mixture containing 150 ml of magnetic particles (3.2 micron, 3.38% w/v with 12% metal oxide content) prepared as described in Example 16, 2 ml of 1% NP 40, 0.5 ml of methyl methacrylate or styrene, 1 g of potassium persulfate and 2 ml of functionalized monomer, trimethylammoniumethyl methacrylate methosulfate (40% aqueous solution), was placed in a sealed bottle. The bottle was rotated at about 60 rpm in a 55^{o}C oven for four hours. The mixture was filtered through two layers of cheese cloth, separated magnetically and washed several times with deionized water until the supernatant was clear. The resulting magnetic particles were resuspended to 200 ml with deionized water to give a 2.5% w/v suspension of magnetic particles with trimethylammonium functional groups on the surface.

### Example 18

Same procedures as described in Example 17 were followed except 1 ml of functionalized monomer, 2-aminoethyl methacrylate, was used to give 200 ml of 2.5% w/v suspension of magnetic particles with amino groups on the surface.

### Example 19

Same procedures as described in Example 17 were followed except 1 ml of functionalized monomer, 2-hydroxyethyl methacrylate, was used to give 200 ml of 2.5% w/v suspension of magnetic particles with hydroxyl groups on the surface.

### Example 20

Same procedures as described in Example 17 were followed except 1 ml of monomer, 1-vinyl-2-pyrrolidinone, was used to give 200 ml of 2.5% w/v suspension of magnetic particles with polyvinylpyrrolidinone on the surface.

### Example 21

Same procedures as described in Example 17 were followed except 1 g of functionalized monomer, methyl propene sulfonic acid, was used to give 200 ml of 2.5% w/v suspension of magnetic particles with sulfonic acids groups on the surface.

### Example 22

Same procedures as described in Example 17 were followed except 1 ml of functionalized monomer, dimethylaminoethyl methacrylate, was used to give 200 ml of 2.5% w/v suspension of magnetic particles with dimethylamino groups on the surface.

### Example 23

A mixture containing 20 ml of 7.0% w/v, 2.11 micron polystyrene particles, 100 ml of 1.8% w/v metal oxide prepared as described in Example 5, 50 ml of deionized water and a solution containing 0.15 g of benzoyl peroxide in 7.5 ml of styrene was placed in a sealed bottle. The bottle was evacuated and rotated at about 60 rpm in a 55^{o}C oven for fifteen hours. The mixture was filtered through two layers of cheese cloth, separated magnetically and washed several times with deionized water until the supernatant was clear. The resulting magnetic particles were resuspended to 200 ml with deionized water to give 5.0% w/v suspension with about 16.8% metal oxide content and 3.6 micron average size.

### Example 24

A mixture containing 20 ml of 7.0% w/v, 2.11 micron polystyrene particles, 100 ml of 1.8% w/v metal oxide prepared as described in Example 5, 50 ml of deionized water and a solution containing 0.15 g of benzoyl peroxide and 0.75 ml of divinyl benzene in 6.75 ml of styrene was placed in a sealed bottle. The bottle was evacuated and rotated at about 60 rpm in a 55^{o}C oven for fifteen hours. The mixture was filtered through two layers of cheese cloth, separated magnetically and washed several times with deionized water until the supernatant was clear. The resulting cross-linked magnetic particles were resuspended to 200 ml with deionized water to give 5.0% w/v suspension with about 16.8% metal oxide content and 3.6 micron average size. The cross-linked magnetic particles prepared this way were found to be uniform in size and inert to common organic solvents such as acetone, acetonitrile and dimethyl formamide.

### Example 25

A mixture containing 20 ml of 7.0% w/v, 2.11 micron polystyrene particles, 150 ml of 1.8% w/v metal oxide prepared as described in Example 5 and a solution containing 0.15 g of benzoyl peroxide, 0.75 ml of divinyl benzene in 6.75 ml of styrene was placed in a sealed bottle. The bottle was evacuated and rotated at about 60 rpm in a 55^{o}C oven for fifteen hours. The mixture was filtered through two layers of cheese cloth, separated magnetically and washed several times with deionized water until the supernatant was clear. The resulting cross-linked magnetic particles were resuspended to 200 ml with deionized water to give 5.4% w/v suspension with about 23% metal oxide content and 4.0 micron average size. The cross-linked magnetic particles prepared this way were found to be uniform in size and inert to common organic solvents such as acetone, actonitrile and dimethyl formamide.

### Example 26

A mixture containing 15 ml of 9.16% w/v, 3.2 micron polystyrene particles, 100 ml of 1.8% w/v metal oxide prepared as described in Example 5, 55 ml of deionized water and a solution containing 0.15 g of benzoyl peroxide and 0.75 ml of divinyl benzene in 6.75 ml of styrene was placed in a sealed bottle. The bottle was evacuated and rotated at about 60 rpm in a 55^{o}C oven for fifteen hours. The mixture was filtered through two layers of cheese cloth, separated magnetically and washed several times with deionized water until the supernatant was clear. The resulting cross-linked magnetic particles were resuspended to 200 ml with deionized water to give 4.7% w/v suspension with about 16.8% metal oxide content and 5.5 micron average size. The cross-linked magnetic particles prepared this way were found to be uniform in size and inert to common organic solvents such as acetone, actonitrile and dimethyl formanide.

### Example 27

A mixture containing 30 ml of 4.5% w/v, 4.1 micron polystyrene particles, 100 ml of 1.8% w/v metal oxide prepared as described in Example 5, 40 ml of deionized water and a solution containing 0.15 g of benzoyl peroxide and 0.75 ml of divinyl benzene in 6.75 ml of styrene was placed in a sealed bottle. The bottle was evacuated and rotated at about 60 rpm in a 55^{o}C oven for fifteen hours. The mixture was filtered through two layers of cheese cloth, separated magnetically and washed several times with deionized water until the supernatant was clear. The resulting cross-linked magnetic particles were resuspended to 200 ml with deionized water to give 4.5% w/v suspension with about 16.9% metal oxide content and 6.7 micron average size. The cross-linked magnetic particles prepared this way were found to be uniform in size and inert to common organic solvents such as acetone, acetonitrile and dimethyl formamide.

### Example 28

A mixture containing 20 ml of 7.0% w/v, 2.11 micron polystyrene particles, 100 ml of 1.8% w/v metal oxide prepared as described in Example 5, 50 ml of deionized water and a solution containing 0.15 g of benzoyl peroxide, 0.75 ml of undecylenyl alcohol and 0.75 ml of divinyl benzene in 6 ml of styrene was placed in a sealed bottle. The bottle was evacuated and rotated at about 60 rpm in a 55^{o}C oven for fifteen hours. The mixture was filtered through two layers of cheese cloth, separated magnetically and washed several times with deionized water until the supernatant was clear. The resulting cross-linked hydroxyl magnetic particles were filtered and dried to give 9 g of powder with about 16.8% metal oxide content and 3.9 micron average size. The cross-linked hydroxyl magnetic particles prepared this way were found to be uniform in size and inert to common organic solvents such as acetone, acetonitrile and dimethyl formamide.

### Coupling Biological Materials to Magnetic Particles

### Example 29

In a 80 ml bottle was placed 30 ml of 4.3 micron, 5.0% w/v carboxyl magnetic particles prepared as described in Example 7. The particles were separated magnetically and resuspended in 50 ml of phosphate buffer (0.1 M, pH 5.5). The the particle suspension were added 20 mg of bovine serum albumin and 1090 mg of 1-ethyl-3-(3-dimethylaminopropyl)-carbodiimdie (EDC). The mixture was rotated end to end at room temperature for two hours and separated magnetically. The particles were washed once with 80 ml of phosphate buffer and resuspended to 75 ml with phosphate buffered saline (0.1 M, pH 7.0) to give a 2.0% w/v suspension.

To couple bovine serum albumin to magnetic particles by passive adsorption the same procedures were followed except no EDC was used.

### Example 30

In a 4 ml vial was placed 1 ml of 4.3 micron, 5.0% w/v carboxyl magnetic particles prepared as described in Example 7. The particles were separated magnetically and washed once with 2 ml of phosphate buffer (0.1 M, pH 5.5) and resuspended to 2 ml with the same buffer. To the particles suspension were added 140 ml of 1.4 mg/ml Goat (Gt) anti Mouse (Ms) IgG and 10 mg of 1-ethyl-3-(3-dimenthylaminopropyl) carbodiimide. The vial was rotated end to end at room temperature for two hours. The particles were separated magnetically, washed once with 2 ml of phosphate buffer and resuspended to 2 ml with phosphate buffered saline (0.1 M, pH 7.0) to give a 2.5% w/v Gt anti MS IgG coated magnetic particles. Other kind of antibody either monoclonal or polyclonal could also be coupled to carboxyl magnetic particles by using the same procedures.

To couple Gt anti Ms IgG or other kind of antibody to the magnetic particles by passive adsorption the same procedures were followed except no EDC was used.

### Example 31

In a 4 ml vial was placed a 2.5 ml of bovine serum albumin coated magnetic particles (4.3 micron, 2% w/v) prepared as described in Example 29. The particles were separated magnetically and resuspended to 2 ml with phosphate buffer (0.1 M, pH 5.5). To the mixture were added 10 ul of Ms anti B red cells surface antigen (20 mg/ml) and 1 mg of 1-ethyl-3-(3-dimethylaminopropyl)carbodiimide. The mixture was rotated end to end at room temperature for two hours. The particles were separated magnetically, washed once with phosphate buffer and resuspended in 2 ml of phosphate buffered saline (0.1 M, pH 7.0) to give a 2.5% w/v suspension.

### Example 32

Same procedures as described in Example 31 were followed except using 40 ul of Ms anti A red cells surface antigen (5 mg/ml) to give 2 ml of 2.5% w/v suspension.

### Blood Typing Using Magnetic Particles

### Example 33

In a 5mm x 65mm test tube labelled A was placed 25 ul of 2.5% w/v Ms anti A coated magnetic particles prepared as described in Example 32. To the other test tube labelled B was placed 25 ul of 2.5% w/v Ms anti B coated magnetic particles prepared as described in Example 31. To both test tubes was added 50 ul of 1% packed red blood cells prepared by 1 to 100 dilution of packed red blood cells in isotonic buffered saline. The test tubes were shaked by finger tapping for several times and placed on the top of a magnet. The results were summarized as follows:

| | BLOOD TYPE | | | |
|---|---|---|---|---|
| | A | B | O | AB |
| Tube A | + | - | - | + |
| Tube B | - | + | - | + |

Where + represent a positive reaction, meaning the red cells were agglutineated by the corresponding antibody coated magnetic particles as a result the supernatant in the test tube was clear after magnetic separation. On the other hand the supernatant of a negative reaction would remain cloudy after magnetic separation due to the absence of agglutination between the red cells and the antibody coated magnetic particles.

### Immunoassays Using Magnetic Particles

### Example 34

In a 2 ml microcentrifuge tube was placed 1 ml of 6% w/v, 3 micron carboxyl magnetic particles. The particles were centrifuged for 3 minutes at 10000 rpm. The supernatant was aspirated and the particles were resuspended by vortexing with 1 ml of 5 to 100 ug/ml recombinant HBcAg in acetate buffer. The tube was rotated at room temperature for two hours and centrifuged as described before. The supernatant was aspirated and the particles were resuspended in 1 ml of overcoat solution containing acetate buffer and 2 to 10% of normal animal serum. The tube was rotated at room temperature for 2 to 16 hours and centrifuged as described before. The supernatant was aspirated and the particles were washed three times with 1 ml of isotonic buffered saline (IBS) by centrifugation and resuspension. Finally, the particles were resuspended with 1 ml of IBS and stored at 2 to 8^{o}C.

### Example 35

To the first two columns of a 96-well microtiter plate was placed 20 ul of 0.25% w/v hepatitis B core antigen (HBcAg) coated magnetic particles prepared as described in Example 34. Sample preparation consisted of various dilutions of a HBcAb positive serum into a negative plasma, followed by a 1:100 dilution of each sample into specimen dilution buffer (SDB). The SDB contained phosphate buffer, protein stabilizers, detergent and antimicrobial agents. To the wells containing the particles were added 50 ul of each final sample dilution. After thirty minutes incubation at 37^{o}C, the particles were separated for two minutes on a magnetic separator and washed three times with 200 ul wash buffer containing slats and detergent. To each well containing thew particles was added 50 ul of goat antihuman IgG-B-D-galactosidase conjugate (0.5 ug/ml) in diluent containing salts, protein stabilizers, glycerol, detergent and antimicrobial agents. After fifteen minutes incubation at 37^{o}C the particles were separated and washed three times as described above and resuspended in 30 ul of IBS. The particles were transferred to the first two columns of a black microtiter plate (Dynatech). To each well containing the particles was added 100 ul of a solution containing 4-methylumbelliferyl-B-galactopyranoside (MUG, Sigma). The plate was incubated at 37^{o} and the fluorescence intensity was measured by using a Fluorescence Concentration Analyzer (FCA, Pandex) equipped with 365 nm excitation and 450 nm emission filters at five minute intervals and 10 X gain setting. The increase in fluorescence intensity in five minute intervals was recorded in arbitrary fluorescence unit (AFU) and presented in Table 1.

**TABLE 1**

| Dilution of Positive Specimen | AFU (5 Minutes) Average of Two Wells |
|---|---|
| 1:100 | 22687 |
| 1:1000 | 5933 |
| 1:5000 | 1516 |
| 1:8000 | 835 |
| 1:10000 | 639 |
| 1:15000 | 495 |
| 1:20000 | 427 |
| 1:25000 | 307 |

### Example 36

The coupling of mouse anti-HBsAg to carboxyl magnetic particles was similar to Example 30.

To the wells of a black 96-well microtiter plate (Dynatech) were added 20 ul of 0.25% w/v, 3.2 micron, mouse anti-HBsAg coated carboxyl magnetic particles in duplicate. To the wells containing the magnetic particles was added 100 ul of neat plasma containing various amounts of HBsAg or a HBsAg-negative plasma. After 30 minutes incubation at 37^{o}C, the particles were separated for two minutes on a magnetic separator and washed once with 100 ul of wash buffer containing slats and detergent. To each well containing the particles was added 20 ul of mouse anti-HBsAg-B-galactosidase conjugate in diluent containing salts, protein stabilizers, glycerol, detergent and antimicrobial agents. After fifteen minutes incubation at 37^{o}C, the particles were separated and washed five times as described above. To each well containing the particles was added 50 ul of a solution containing 4-methylumbelliferyl-B-D-galactopyranoside (MUG, Sigma). The plate was incubated at 37^{o}C and the fluorescence intensity was measured by using a Fluorescence Concentration Analyzer (FCA, Pandex) equipped with 365 nm excitation and 450 nm emission filters at five minute intervals and 10 X gain setting. The increase in fluorescence intensity in five minute intervals was recorded in arbitrary fluorescence unit (AFU) and presented in Table 2.

**TABLE 2**

| HBsAg Conc. (nano gm) | AFU (5 Minutes) Average of Two Wells |
|---|---|
| 1.0 | 1149 |
| 0.5 | 455 |
| 0.25 | 218 |
| 0.125 | 118 |
| neg. | 14 |

### Example 37

The HIV-1 antigens from HTLV-IIIB/H-9 cells (Gallo Strain) were coupled to 3.6 micron carboxyl magnetic particles by using similar procedures as described in Example 34.

To the wells of a 96-well microtiter plate were added 20 ul of 0.25% w/v of HIV coated magnetic particles in duplicate. To the wells containing the particles were added 50 ul of positive, borderline and negative specimens diluted 1:100 in specimen dilution buffer (SDB) containing phosphate buffer, protein stabilizers, detergent and antimicrobial agents. After thirty minutes incubation at 37^{o}C, the particles were separated for two minutes on a magnetic separator and washed three times with 100 ul of washed buffer containing salts and detergent. To each well containing particles was added 50 ul of goat antihuman-B-galactosidase (approximately 0.5 ug/ml) conjugate in diluent containing salts, protein stabilizers, glycerol, detergent and antimicrobial agents. After fifteen minutes incubation at 37^{o}C, the particles were washed four times as described above. The particles were transferred to the black microtiter plate (Dynatech). To each well containing particles was added 100 ul of a solution containing 4-methylumbelliferyl-B-D-galactopyranoside (MUG, Sigma). The plate was incubated at 37^{o}C and the fluorescence intensity was measured by using a Fluorescence Concentration Analyzer (FCA, Pandex) equipped with 365 nm excitation and 450 nm emission filters at five minute intervals and 25 X gain setting. The increase in fluorescence intensity in a five minute interval was recorded in arbitrary fluorescence unit (AFU) and presented in Table 3.

**TABLE 3**

| Anti-HIV Specimens | AFU (5 Minutes) Average of Two Wells |
|---|---|
| Positive Control | 9462 |
| Borderline Specimen | 527 |
| Negative Control | 86 |

### Cell Separation Using Magnetic Particles

### Example 38

The 4.3 micron carboxyl magnetic particles prepared as described in Example 7 were washed and sonicated in phosphate buffered saline (PBS, pH7.7), sterilized in 70% ethanol for 10 minutes, washed three times in PBS and incubated for 48 hours at 4^{o}C with affinity-purified sheep antimouse immunoglobulin antibody (SAM) at 0.5 mg/ml and a ratio of 3.3 mg antibody/100 mg particles. Before use, the antibody coated magnetic particles were washed in PBS and resuspended at the desired concentration in PBS.

Human tissue culture cALLa-positive NALM-16 leukemia cells were washed and suspended in PBS. One fraction was not treated with antibody (-MoAb). The other fraction was treated with two anti-CD10 and one anti-CD9 monoclonal antibodies (+MoAb) for thirty minutes at 4^{o}C, washed in PBS and adjusted to 3.5 x 10⁶ cells/ml on PBS. To two tubes, one containing the antibody treated cells (+MoAb), the other containing untreated cells (MoAb) were added SAM coated magnetic particles at a particle to starting cell ratio of 45. The tubes were rotated at 4^{o}C for thirty minutes. The particles were separated with a magnetic separator. The supernatant was collected and centrifuged to collect the remaining cells. The pellet was resuspended in 100 ul of trypan blue and total cell count was made. The results were presented in Table 4.

**TABLE 4**

| Particle/cell Ratio | Cells +/- MoAb | Cells Received | % Depletion |
|---|---|---|---|
| 0 | + | 7.62x10⁵ | 0 (Control) |
| 45 | + | 2.89x10⁴ | 96.2 |
| 45 | - | 7.33x10⁵ | 4.6 |

### Example 39

The paramagnetic particles of the present invention were compared to particles from commercial sources as follows: Pfaltz & Bauer, ferro ferric oxide suspension Average size: less than 2 um, pH7; Pfaltz & Bauer, ferro ferric oxide suspension Average size: less than 2 um, pH 11; Ferro Fluides, iron oxide suspension Average size: less than 1 um.

A mixture containing 64 ml of deionized water, 32 ml of 3.0% w/v magnetic metal oxide, and 1.0 ml of styrene was placed in a sealed bottle. The bottle was evacuated and rotated at about 60 rpm in a 60^{o}C oven for one hour. To the mixture were added 47 ml of 10% 4 um polystyrene particles and 2.0 grams of potassium persulfate. The bottle was resealed, evacuated and rotated in a 60^{o}C oven for one hour after which 5.0 ml of 2% sodium dodecylsulfate was added. After five more hours 1.0 ml of styrene and 1.0 gram of potassium persulfate were added. The mixture was rotated for another fifteen hours, filtered through two layers of cheese cloth, and examined under the microscope.

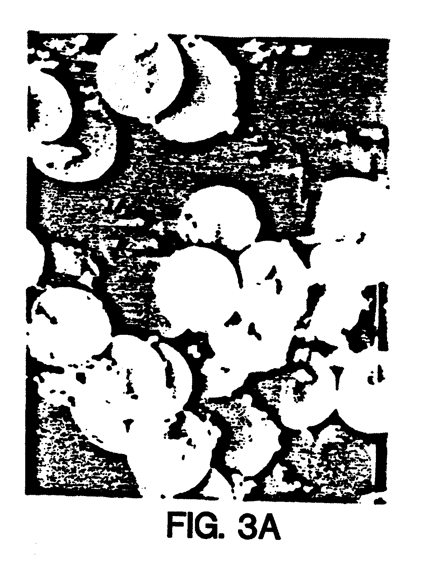

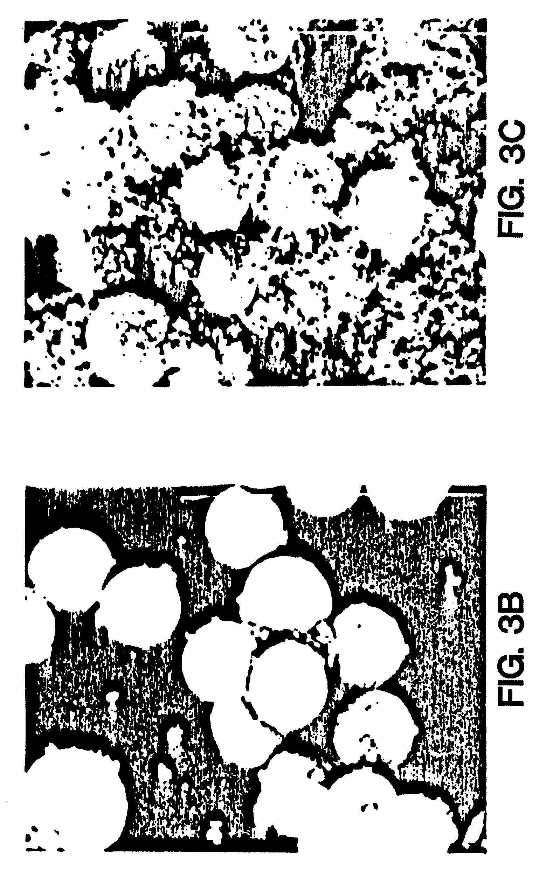

In all cases, the particles coated poorly to the core particles compared to the control utilizing magnetic iron oxide particles made according to our alkali precipitation method. There was also substantial aggregation of the commercial particles. The photomicrographs of the particles prepared according to this experiment are attached below. Figures 3a to 3c correspond to the three types of beginning materials listed above, progressing from A to C.

### Example 40

In this experiment, the reaction products of the present process were compared in the presence and absence of the core particles. The same procedure was employed as in Example 39, only omitting the core particles in one reaction.

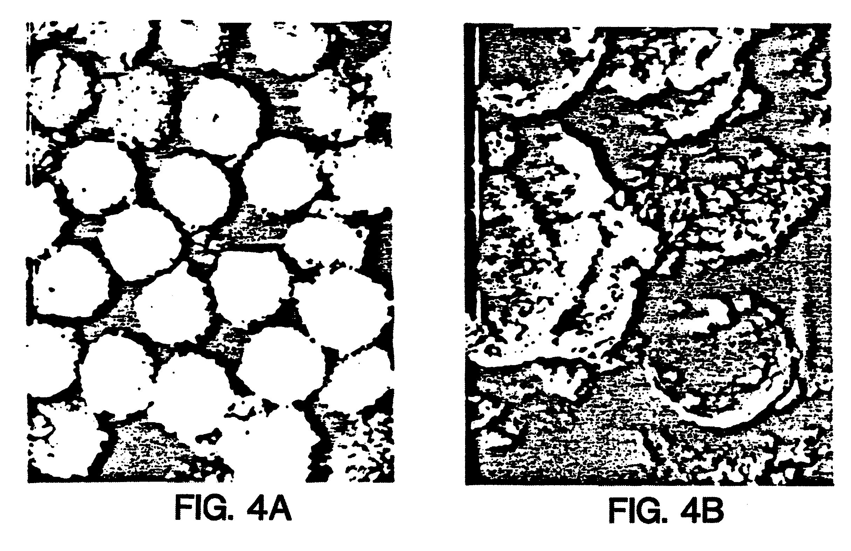

The control (with core particles) gave excellent coated particles, as shown in the photomicrograph of Figure 4a. The sample generated without the core particles shows a great deal of aggregation (Figure 4b), indicating that styrene readily migrates into the particle phase. The results also suggest that the coating of particles is essential one step, in that adhesion of magnetic iron oxide particles to the core particle occurs quickly enough that aggregation is effectively prevented. One would normally expect these to be competing reactions, with the accumulation of a significant amount of aggregates. It is therefore surprising that more than 90% of the magnetic particles coat instead of aggregate.

### Example 41

In this experiment, the exclusion limits of the amount of styrene that can be added to create magnetic particles was tested. We calculated the amount of styrene needed to enlarge a core particle of about 2.8 um to one of about 4 u.

A mixture containing 27 ml of 2.5% w/v metal oxide, 40 ml of 10% 2.8 um polystyrene particles, 10.8 ml of styrene, 0.96 gram of potassium persulfate and 74 ml of deionized water was placed in a sealed bottle. The bottle was evacuated and rotated at 60 rpm in a 60^{o}C oven for 16 hours, and examined under the microscope.

The results indicate that use of an excess of styrene causes massive aggregation into a single large clump separated from a milky suspension of < 1 um size of polystyrene particles, indicating that the weight ratio parameters are important in obtaining proper coating using ferro ferric metal oxide. These results also indicate that the use of a core particle is essential to uniform deposition of magnetic material, and only incidentally yields a particles with potentially lower density than one composed entirely of magnetic material, or one through which the magnetic material is fully dispersed.

### Example 42

In this experiment, oleophilic magnetic iron oxide particles were first prepared utilizing both our particles made by alkali precipitation, and particles obtained from a commercial source. The standard coating procedure was then carried out in the presence of the core particles.

Fourteen ml of 20% w/w aqueous potassium oleate solution was added to 34 ml of water containing 3.4 grams of each iron oxide particle. The resulting mixture was stirred at 90^{o}C for 30 minutes. After cooling the mixture, the pH was adjusted to 6 with O, 5N, HCl. The agglomerated particles were collected by separating on a magnetic separator, washed with two 10 ml portions of water at 80^{o}C, followed by two 10 ml portions of ethanol, and dried under reduced pressure.

Using the metal oxides so prepared, 0.96 gram of oleophilic particles was dispersed in 1 ml of styrene. To this dispersed solution was added 64 ml of deionized water. The mixture was sonicated for 10 minutes to aid dispersion, and the bottle was sealed. The rest of the procedure is the same as for Example 39 above.

Using the oleophilic particles derived from the alkali precipitated material, a small amount of coating was observed. However, considerable aggregating of core particles is seen, as well as substantial amounts of aggregated magnetic metal oxide particles. In the case of the oleophilic commercial iron oxide particles, there is aggregation of metal oxides to a clump and aggregation of the core particles. The results indicate that the present method will not work when the magnetic particles are made compatible with the organic phase monomer.

### Example 43

This experiment compares the amount of passive adsorption of a peptide antigen (avidin) to polystyrene particles, polystyrene particles have a bumpy external layer containing magnitite made according to the present method, and polystyrene particles having a smooth external layer containing the present magnitite. The particles all were made to the same final diameter.

10ml of 2.5% w/v of each type of particle were pelleted by centrifugation at 2000rpm for 10 minutes. The supernatant was removed and the pellet was resuspended in 10 ml of 0.1M phosphate buffer, pH 5.5. The particles were repelleted and the supernatant removed.

Lyophilized avidin was suspended in 0.1M phosphate buffer pH 5.5 at a concentration of 0.5mg/ml. 4 ml of the peptide solution was then transferred to each particle pellet. The particles were resuspended in the peptide solution and tumbled for 12 hours at room temperature. The passively adsorbed avidin coated particles were then pelleted at 2000 rpm. The supernatant was carefully collected. The particles were washed twice more, and the supernatants added to the first. The supernatants were then filtered through a 0.2nm filter and the optical density was measured. The results were as follows:

| | O.D. |
|---|---|
| Plain polystyrene particles | .6103 |
| Smooth polystyrene particles | .4717 |
| Bumpy polystyrene particles | .4380 |
| 0.5% avidin control | .7103 |

The data indicate that both the bumpy and smooth-surfaced particles containing an outer layer of core particle surface-polymerized polystyrene in which magnitite comprising small crystals bound together by amorphous iron oxide precipitate matter is embedded, exhibit a high degree of peptide adsorption compared to plain polystyrene particles of the same size.

The virtually identical coating of smooth particles, made with sufficient monomer to ensure complete embedding of magnitite, to the coating of the rough particles, suggests that the greater capacity is not due mainly to surface area or surface topology. Applicants have no explanation of this phenomenon.

## Claims

1. A paramagnetic microparticle comprising
an inner polymeric core particle 1 to 100 microns in diameter; and an external layer containing metal oxide crystals,
characterised in that the external layer is a polymeric layer polymerized at the surface of said core particle, said metal oxide crystals being agglomerated with an amorphous metal oxide precipitate, into nonuniformly sized clusters of about 1.0 microns or less.

2. The microparticle of claim 1 wherein the said polymeric core particle and the external polymeric layer are polystyrene.

3. The microparticle of claim 1 or 2 wherein said clusters comprising metal oxide crystals agglomerated with an amorphous metal oxide precipitate are produced by heating an aqueous solution containing a mixture of divalent and trivalent transition metal salts in a molar ratio of divalent to trivalent metal salt of 0.5 to 2.0 under alkaline conditions to produce precipitation, washing the said clusters, breaking down the aggregate of metal oxide crystals, and collecting the smaller particles of a size less than 1 micron.

4. A process to determine the presence or concentration of an analyte comprising
coating the microparticles of claim 1 2, or 3 with a ligand specific for said analyte
contacting said coated microparticles with an aqueous solution containing said analyte
incubating the said solution
separating said microparticles from said solution
adding a second labelled ligand specific for said analyte to said microparticles contained in an aqueous solution to suspend said microparticles
incubating the said solution
separating said microparticles from said solution
measuring the amount of labelled ligand associated with said microparticles.

5. A process to determine the presence or concentration of specific nucleic acid sequences in nucleic acid target molecules comprising
attaching to the microparticles of claim 1 2, or 3 a nucleic acid complementary to said nucleic acid sequence of said target molecule;
contacting said microparticles with a fluid specimen containing said complementary nucleic acid to form a suspension
incubating said suspension under hybridizing conditions for a period of time sufficient to permit hybridization
separating said microparticles from said suspension
adding a second labelled nucleic acid having a complementary sequence to said target different than that of the sequence of the nucleic acid attached to said microparticles
incubating said suspension under hybridizing conditions for a period sufficient to permit hybridization
separating said microparticles from said solution
detecting duplex formation on said microparticle by measuring said label.

6. A process for removing an unwanted biosubstance comprising
coating a ligand specific for said biosubstance to the microparticles of claim 1, 2 or 3
contacting said microparticles with a solution containing said biosubstance to form a suspension
incubating said suspension until said biosubstance has reacted with said ligand, and
separating said microparticles from said suspension.

## Patentansprüche

1. Paramagnetisches Mikropartikel, das aufweist:
ein inneres polymeres Kernpartikel mit einem Durchmesser von 1 bis 100 »m; und eine äußere Schicht, die Metalloxidkristalle enthält,
dadurch gekennzeichnet, daß die äußere Schicht eine polymere Schicht ist, die an der Oberfläche des Kernpartikels polymerisiert ist, wobei die Metalloxidkristalle mit einem amorphem Metalloxid-Präzipitat zu ungleichmäßig großen Clustern von ca. 1,0 »m oder kleiner agglomeriert sind.

2. Mikropartikel nach Anspruch 1, wobei das polymere Kernpartikel und die äußere polymere Schicht Polystyrol sind.

3. Mikropartikal nach Anspruch 1 oder 2, wobei die Cluster, die Metalloxidkristalle aufweisen, die mit einem amorphen Metalloxid-Präzipitat agglomeriert sind, hergestellt sind durch Erhitzen einer wäßrigen Lösung, die ein Gemisch aus zwei- und dreiwertigen Übergangsmetallsalzen in einem Molverhältnis von zweiwertigem zu dreiwertigem Metallsalz von 0,5 bis 2,0 enthält, unter alkalischen Bedingungen, um Präzipitieren zu verursachen, Waschen der Cluster, Aufspalten des Aggregats von Metalloxidkristallen und Auffangen der kleineren Partikel einer Größe von weniger als 1 »m.

4. Verfahren zum Bestimmen der Anwesenheit oder Konzentration eines Analyten, wobei das Verfahren folgendes aufweist:
Beschichten der Mikropartikel von Anspruch 1, 2 oder 3 mit einem für diesen Analyten spezifischen Liganden,
In-Kontakt-Bringen der beschichteten Mikropartikel mit einer wäßrigen Lösung, die den Analyten enthält,
Inkubieren der Lösung,
Trennen der Mikropartikel von der Lösung,
Zugeben eines zweiten, markierten Liganden, der für den Analyten spezifisch ist, zu den in einer wäßrigen Lösung enthaltenen Mikropartikeln, um die Mikropartikel zu suspendieren,
Inkubieren dieser Lösung,
Trennen der Mikropartikel von der Lösung,
Messen der Menge von markiertem Liganden, die mit den Mikropartikeln assoziiert ist.

5. Verfahren zum Bestimmen der Anwesenheit oder Konzentration von spezifischen Nucleinsäure-Sequenzen in Nucleinsäure-Targetmolekülen, wobei das Verfahren folgende Schritte aufweist:
Anheften einer Nucleinsäure, die zu der Nucleinsäure-Sequenz des Targetmoleküls komplementär ist, an die Mikropartikel von Anspruch 1, 2 oder 3;
In-Kontakt-Bringen der Mikropartikel mit einer flüssigen Probe, die die komplementäre Nucleinsäure enthält, um eine Suspension zu bilden;
Inkubieren der Suspension unter Hybridisierungsbedingungen für einen Zeitraum, der ausreicht, um die Hybridisierung zuzulassen;
Trennen der Mikropartikel von der Suspension;
Zugeben einer zweiten markierten Nucleinsäure, die eine zu dem Target komplementäre Sequenz hat, die von derjenigen der an die Mikropartikel angehefteten Nucleinsäure-Sequenz verschieden ist;
Inkubieren der Suspension unter Hybridisierungsbedingungen für einen Zeitraum, der ausreicht, um die Hybridisierung zuzulassen;
Trennen der Mikropartikel von der Lösung;
Nachweisen der Doppelstrangbildung an dem Mikropartikel durch Messen des Markers.

6. Verfahren zum Entfernen einer unerwünschten Biosubstanz, wobei das Verfahren folgende Schritte aufweist:
Beschichten eines für die Biosubstanz spezifischen Liganden mit den Mikropartikeln der Ansprüche 1, 2 oder 3,
In-Kontakt-Bringen der Mikropartikel mit einer die Biosubstanz enthaltenden Lösung, um eine Suspension zu bilden,
Inkubieren der Suspension, bis die Biosubstanz mit dem Liganden reagiert hat, und
Trennen der Mikropartikel von der Suspension.

## Revendications

1. Microparticule paramagnétique comprenant une particule noyeau polymérique interne de 1 à 100 microns de diamètre ; et une couche externe contenant des cristaux d'oxyde métallique, caractérisée en ce que la couche est une couche polymérique polymérisée à la surface de ladite particule noyau, lesdits cristaux d'oxyde métallique étant agglomérés avec un précipité d'oxyde métallique amorphe, en amas de taille non uniforme d'environ 1,0 microns ou moins.

2. Microparticule de la revendication 1 dans laquelle ladite particule noyeau polymérique et la couche polymérique externe sont du polystyrène.

3. Microparticule de la revendication 1 ou 2 dans laquelle lesdits amas comprenant des cristaux d'oxyde métallique agglomérés avec un précipité d'oxyde métallique amorphe sont produits en chauffant une solution aqueuse contenant un mélange de sel de métaux de transition bivalents et trivalents dans un rapport molaire du sel de métal bivalent au sel de métal trivalent de 0,5 à 2,0 dans des conditions alcalines pour produire une précipitation, en lavant lesdits amas, en brisant les agrégats de cristaux d'oxyde métallique, et en recueillant les petites particules d'une taille inférieure à 1 micron;

4. Procédé pour déterminer la présence ou la concentration d'un analyte dans lequel
on revêt les microparticules de la revendication 1, 2 ou 3 avec un coordinat spécifique dudit analyte,
on met en contact lesdites microparticules revêtues avec une solution aqueuse contenant ledit analyte,
on fait incuber ladite solution,
on sépare lesdites microparticules de ladite solution,
on ajoute un second coordinat marqué spécifique dudit analyte auxdites microparticules contenues dans une solution aqueuse pour mettre en suspension lesdites microparticules,
on fait incuber ladite solution,
on sépare lesdites microparticules de ladite solution,
on mesure la quantité de coordinat marqué associée auxdites microparticules.

5. Procédé pour déterminer la présence ou la concentration de séquences d'acides nucléiques spécifiques dans des molécules cibles d'acide nucléique dans lequel
on attache aux microparticules de la revendication 1, 2 ou 3 un acide nucléique complémentaire de ladite séquence d'acide nucléique de ladite molécule cible ,
on met en contact lesdites microparticules avec un échantillon de fluide contenant ledit acide complémentaire pour former une suspension ,
on fait incuber ladite suspension dans des conditions hybridantes pendant une durée suffisante pour permettre l'hybridation,
on sépare lesdites microparticules de ladite suspension,
on ajoute un second acide nucléique marqué ayant une séquence complémentaire de ladite cible différente de celle de la séquence de l'acide nucléique attaché auxdites microparticules,
on fait incuber ladite suspension dans des conditions hybridantes pendant une durée suffisante pour permettre l'hybridation,
on sépare lesdites microparticules de ladite solution,
on détecte la formation d'un duplex sur ladite microparticule en mesurant ladite marque .

6. Procédé pour enlever une biosubstance indésirable dans lequel
on dépose un coordinat spécifique de ladite biosubstance sur les microparticules des revendications 1 , 2 ou 3,
on met en contact lesdites microparticules avec une solution contenant ladite biosubstance pour former une suspension,
on fait incuber ladite suspension jusqu'à ce que ladite biosubstance ait réagi avec ledit coordinat, et
on sépare lesdites microparticules de ladite suspension.
